# EUROPEAN PATENT SPECIFICATION

(11) **EP 1 747 030 B1**
(45) Date of publication and mention of the grant of the patent: **24.07.2013**
(21) Application number: 05749746.3
(22) Date of filing: 19.05.2005
(51) Int. Cl.: A61L 27/30, A61L 27/44, A61L 31/08, A61L 31/12, A61L 29/10, A61L 29/12

(54) **MEDICAL DEVICES AND METHODS OF MAKING THE SAME**
MEDIZINPRODUKTE UND VERFAHREN ZU IHRER HERSTELLUNG
DISPOSITIFS MEDICAUX ET PROCEDES DE FABRICATION DE CEUX-CI

(30) Priority: 20.05.2004 US 850085
(43) Date of publication of application: 31.01.2007
(73) Proprietor: Boston Scientific Limited, Barbados, West Indies (BB)
(72) Inventor: WEBER, Jan, Maple Grove, MN 55311 (US); HOLMAN, Thomas, J., Princeton, MN 55371 (US)
(74) Representative: Peterreins, Frank
(86) International application number: PCT/US2005/017565
(87) International publication number: WO 2005/113033

(56) References cited:
- WO-A-01/68158
- US-A- 5 558 903
- US-A1- 2002 001 620
- US-A1- 2003 065 355
- US-A1- 2003 093 107
- US-B1- 6 312 768
- KRASHENINNIKOV A V ET AL: "Production of defects in supported carbon nanotubes under iron irradiation" PHYSICAL REVIEW, B. CONDENSED MATTER, AMERICAN INSTITUTE OF PHYSICS. NEW YORK, US, vol. 65, 15 April 2002 (2002-04-15), pages 165423-1-165423-8, XP002973991 ISSN: 0163-1829
- KRASHENINNIKOV A V ET AL: "Ion-irradiation-induced welding of carbon nanotubes" PHYSICAL REVIEW B (CONDENSED MATTER AND MATERIALS PHYSICS) APS THROUGH AIP USA, vol. 66, no. 24, 15 December 2002 (2002-12-15), pages 245403-1-245403-6, XP002352629 ISSN: 0163-1829 cited in the application
- KRASHENINNIKOV A V ET AL: "Formation of ion-irradiation-induced atomic-scale defects on walls of carbon nanotubes" PHYSICAL REVIEW B (CONDENSED MATTER AND MATERIALS PHYSICS) APS THROUGH AIP USA, vol. 63, no. 24, 15 June 2001 (2001-06-15), pages 245405/1-6, XP002352630 ISSN: 0163-1829 cited in the application
- CHOI E S ET AL: "Enhancement of thermal and electrical properties of carbon nanotube polymer composites by magnetic field processing" JOURNAL OF APPLIED PHYSICS, AMERICAN INSTITUTE OF PHYSICS. NEW YORK, US, vol. 94, no. 9, 1 November 2003 (2003-11-01), pages 6034-6039, XP012060598 ISSN: 0021-8979 cited in the application
- BAHR ET AL: "Functionalization of Carbon Nanotubes by Electrochemical Reduction of Aryl Diazonium Salts: A Bucky Paper Electrode" JOURNAL OF THE AMERICAN CHEMICAL SOCIETY, AMERICAN CHEMICAL SOCIETY, WASHINGTON, DC, US, vol. 123, 14 June 2001 (2001-06-14), pages 6536-6542, XP002298712 ISSN: 0002-7863 cited in the application

## Description

### TECHNICAL FIELD

The invention relates to medical devices and methods of making the devices.

### BACKGROUND

The body includes various passageways such as arteries, other blood vessels, and other body lumens. These passageways, such as a coronary artery, sometimes become constricted or blocked (for example, by plaque). When this occurs, a constricted passageway can be widened in an angioplasty procedure using a balloon catheter, which includes a medical balloon carried by a catheter shaft.

In an angioplasty procedure, the balloon catheter can be used to treat a stenosis, or a narrowing of the body vessel, by collapsing the balloon and delivering it to a region of the vessel that has been narrowed to such a degree that fluid (e.g., blood) flow is restricted. The balloon can be delivered to a target site by passing the catheter shaft over an emplaced guidewire and advancing the catheter to the site. In some cases, the path to the site can be rather tortuous and/or narrow. Upon reaching the site, the balloon is then expanded, e.g., by injecting a fluid into the interior of the balloon. Expanding the balloon can expand the stenosis radially so that the vessel can permit an acceptable rate of fluid flow. After use, the balloon is collapsed, and the catheter is withdrawn.

In some cases, re-stenosis, which is the renarrowing of the vessel, can occur after an angioplasty procedure. To treat restenosis, the vessel can be reopened or reinforced, or even replaced, with a medical endoprosthesis. An endoprosthesis is typically a tubular member that is placed in a lumen in the body. Examples of endoprosthesis include stents, covered stents, and stent-grafts.

Endoprostheses can be delivered inside the body by a balloon catheter that supports the endoprosthesis in a compacted or reduced-size form as the endoprosthesis is transported to a treatment site. The balloon can be inflated to deform and to fix the expanded endoprosthesis at a predetermined position in contact with the vessel wall. The balloon can then be deflated, and the catheter withdrawn.

During angioplasty, stent placement, or other percutaneous methods, plaque, thrombus or other material (e.g., from a lesion) may break loose and drift along the vessel. Under some circumstances, such as when these procedures are performed on saphenous vein grafts, embolism may result from the breaking off of thrombus. To reduce the occurrence of embolism, an intravascular filter can be placed within the body vessel, for example, distally of the treatment site. The filter can be used to filter plaque, thrombus and other debris released into the blood stream treatment, and subsequently be removed.

WO 01/68158 A1 relates to a coating that promotes endothelial cell adherence and which in one embodiment is composed of fullerenes.

### Summary

The invention relates to medical devices and methods of making the devices as set forth in the claims.

In one aspect, the invention features a method of making a medical device that includes a structure including carbon nanotubes, the method (46) of making the structure comprising: providing a mixture containing nanotubes; forming a layer containing nanotubes; forming covalent bonds between carbon nanotubes; forming polymer layers on nanotube-containing layers; and incorporating the structure including nanotubes into the medical device.

Embodiments may include the following features. The structure can be a membrane. The method can further comprise removing solvent from the mixture. The method can further repeating the steps of forming a layer, optionally removing the solvent, and forming bonds between carbon nanotubes. The method can further comprise modifying the nanotubes. The bonds can consist essentially of carbon atoms. Forming bonds can comprise irradiating the carbon nanotubes, e.g. with ions or electrons. The method can further comprise contacting the nanotubes with a polymer or functionalizing the nanotubes. The method can further comprise aligning the nanotubes, e.g. magnetically. The method can further comprise wrapping a plurality of nanotubes with polymer. The nanotubes can comprise single-walled carbon nanotubes.

In another aspect, the invention features medical devices that include a structure including carbon nanotubes, the structure comprising a layer comprising a polymer; the layer comprising carbon nanotubes covalently bonded with other carbon nanotubes. The resulting structure can be relatively strong and tough, while also being thin and flexible.

Embodiments may include the following features. The structure can be a membrane. The carbon nanotubes can be bonded by a bond consisting essentially of carbon atoms. The layer carbon nanotubes can be corrugated. The carbon nanotube can be wrapped with a polymer. The carbon nanotubes can be are aligned. The carbon nanotubes can comprise an organic functional group bonded to the nanotubes. The carbon nanotubes can comprise single-walled carbon nanotubes. The device can be an intravascular filter, a medical balloon, a stent graft, a catheter or a catheter sheath.

Other aspects, features and advantages of the invention will be apparent from the description of the preferred embodiments and from the claims.

### DESCRIPTION OF DRAWINGS

Fig. 1 is an illustration of an intravascular filter.
Fig. 2 is a cross-sectional diagram of a membrane of the filter of Fig. 1, taken along line 2-2.
Fig. 3 is a detailed illustration of a portion of the membrane of Fig. 2.
Fig. 4 is a flow chart of a method of making a nanotube-containing structure.
Fig. 5 is an illustration of a nanotube-containing structure.
Fig. 6 is an illustration of a stent graft.
Fig. 7 is an illustration of a balloon catheter.

### DETAILED DESCRIPTION

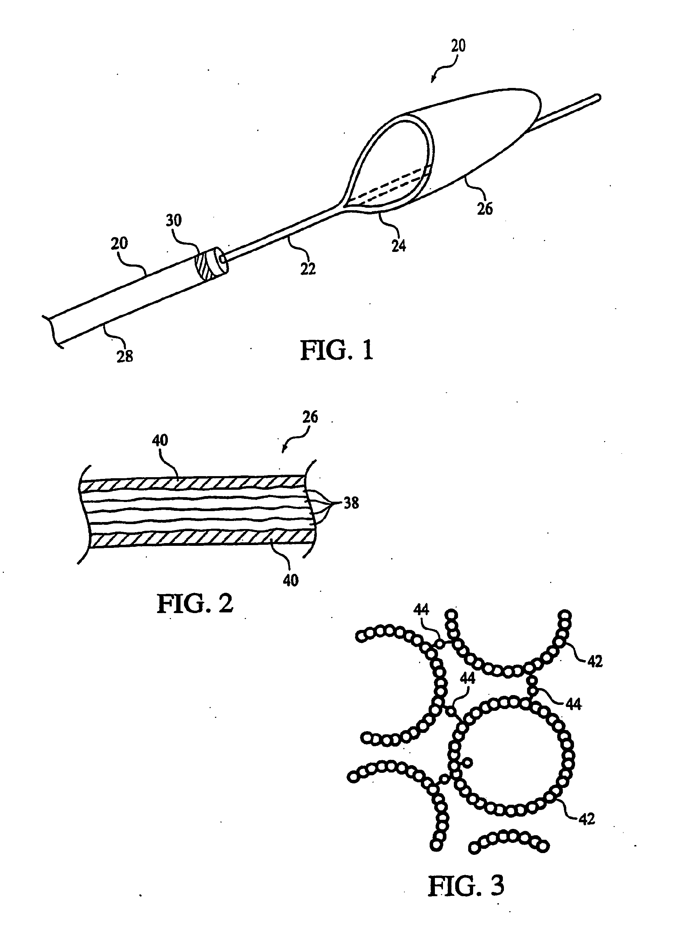

Referring to Fig. 1, an intravascular filter 20 includes a support shaft 22, a deformable frame 24 carried by the support shaft, and a membrane 26 supported by the support shaft and the frame. Membrane 26 includes a plurality of openings (not shown) extending through the membrane to allow bodily fluid to pass through the membrane. Membrane 26 can be connected to shaft 22 and frame 24, for example, by an adhesive or by solvent casting methods. During use, filter 20 can be delivered to and from a target site through a catheter 28 having a radiopaque band 30. Intravascular filters are further described, for example, in Daniel et al., U.S. Patent No. 6,171,327, and exemplified by the FilterWire EX™ Embolic Protection System, available from Boston Scientific Corporation.

Membrane 26 includes carbon nanotubes, such as single-walled carbon nanotubes and multiwalled carbon nanotubes. Referring to Fig. 2, membrane 26 includes one or more (as shown, four) relatively porous, nanotube-containing layers 38, sometimes called "bucky paper" or "nanotube paper", between two or more polymer layers 40. Polymer layer(s) 40 can enhance the strength of membrane 26. Membrane 26 includes one polymer layer (an outer layer or an inner layer). Each nanotube-containing layer 38 includes a mat of entangled carbon nanotubes. In preferred embodiments, referring to Fig. 3, nanotube-containing layer 38 includes nanotubes 42 that are chemically bonded to adjacent nanotubes, for example, by irradiating the nanotubes with ions, such as argon, and/or electrons. The bonds 44, such as covalent carbon-carbon bonds, serve as molecular links that directly connect nanotubes 42 to form a continuous, three-dimensional structure, and to enhance the mechanical properties, such as strength, of nanotube-containing layer 38.

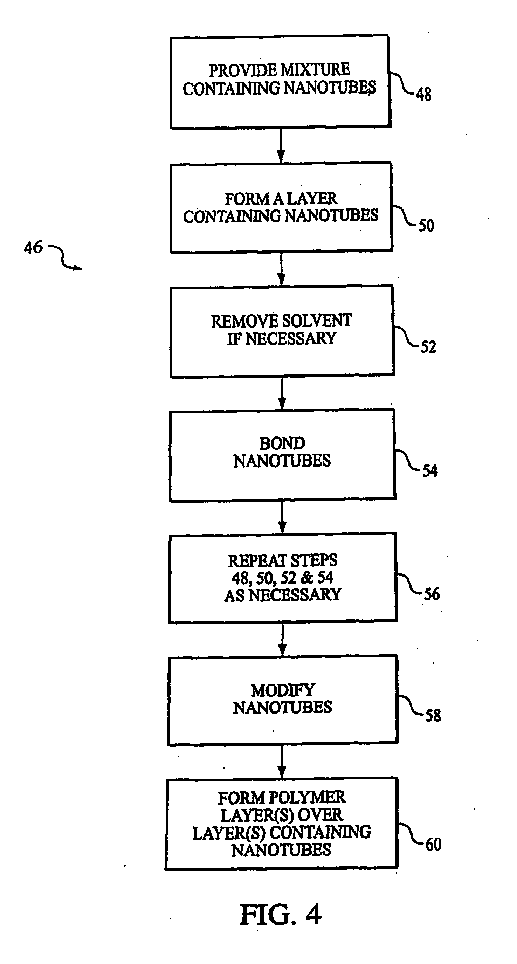

Referring to Fig. 4, a method 46 of making membrane 26 is shown. Method 46 includes providing a mixture containing nanotubes (step 48), and forming a layer containing nanotubes (step 50), for example, by casting the mixture on an appropriately shaped substrate. Next, solvent from the mixture is removed from the formed layer to leave a layer of nanotubes on the substrate (step 52). Inter-nanotube bonds are then formed in the layer of nanotubes (step 54), for example, by irradiating the nanotubes. Next, steps 50, 52, and 54 are repeated as desired to build layer 38 of a predetermined thickness (step 56). As described below, in some embodiments, the nanotubes are functionalized with one or more chemical moieties, for example, to enhance compatibility and/or adhesion with polymer layers 40 (step 58). Polymer layers 40 are formed on nanotube-containing layers 38, for example, by injection molding (step 60). Other embodiments of membrane 26, such as a plurality of alternating nanotube-containing layers and polymer layers, can be formed.

The mixture containing nanotubes includes a suspension of carbon nanotubes in a solvent. The nanotubes include bioinert, hollow single-walled carbon nanotubes (SWNTs) and/or bioinert, hollow multiwalled carbon nanotubes (sometimes called "bucky tubes") having at least one dimension less than about 1000 nm.

The physical dimensions of the nanotubes can be expressed as units of length and/or as a length to width aspect ratio. The nanotubes can have an average length of from about 0.1 micron to about 20 microns. For example, the length can be greater than or equal to about 0.1 micron, 0.5 micron; 1 micron, 5 microns, 10 microns, or 15 microns; and/or less than or equal to about 20 microns, 15 microns, 10 microns, 5 microns, 1 micron, or 0.5 micron. The nanotubes can have an average width or diameter of from about 0.5 nm to about 150 nm. For example, the width or diameter can be greater than or equal to about 0.5 nm, 1 nm, 5 nm, 10 nm, 25 nm, 50 nm, 75 nm, 100 nm, or 125 nm; and/or less than or equal to about 150 nm, 125 nm, 100 nm, 75 nm, 50 nm, 25 nm, 10 nm, 5 nm, or 1 nm. Alternatively or in addition, the nanotubes can be expressed as having a length to width aspect ratio of from about 10:1 to about 50,000:1. The length to width aspect ratio can be greater than or equal to about 10:1, 100:1, or 1,000:1; 2,500:1; 5,000:1; 10,000:1; 20,000:1; 30,000:1; or 40,000:1; and/or less than or equal to about 50,000:1; 40,000:1; 30,000:1; 20,000:1; 10,000:1; 5,000:1; 2,500:1; 1,000:1, or 100:1. The nanotubes preferably have long lengths and small diameters.

The nanotubes are commercially available or they can be synthesized. Carbon nanotubes are available, for example, in a mixture from Rice University (Houston, TX). Synthesis of carbon nanotubes is described, for example, in Bronikowski et al., J. Vac. Sci. Technol. A, 19(4), 1800-1805 (2001); and Davis et al., Macromolecules 2004, 37, 154-160. Dispersion of carbon nanotubes in solvents, for example, to form a film, is described in Ausman et al., J. Phys. Chem. B, 2000, 104(38) 8911-8915; Sreekumar et al., Chem. Mater. 2003, 15, 175-178

To form a layer or mat of nanotubes, sometimes called "bucky paper" (step 50), the nanotubes in the mixture can be separated from the solvent by filtration. For example, approximately four grams of a 0.6 mg/ml nanotube suspension, which can be further diluted by about 80 ml of deionized water, can be vacuum filtered through a polytetrafluoroethylene (PTFE) filter (Millipore LS) or a Whatman Anodisc 47 filter (20 nm pore size). The filtered nanotubes can be washed with 2 x 100 ml of deionized water and 100 ml of methanol. The washed nanotubes can then dried under vacuum at 70°C for twelve hours to remove any residual solvent (step 52) to yield a flexible mat of aggregated nanotubes. In some embodiments, the mat of nanotubes is from about 15 to about 35 microns thick, with a bulk density of about 0.3-0.4 g/cc. In other embodiments, the mat can have a thickness as little as two contacting nanotubes.

Next, bonds are formed between the nanotubes in the mat (step 54). The bonds can be formed by irradiating the nanotubes with ions, such as argon ions or carbon ions, and/or electrons, such as in a transmission electron microscope. Without wishing to be bound by theory, it is believed that when particles of high enough energies collide with carbon atoms, the incident particles can displace atoms in the nanotubes, and form defects, such as vacancies, and dangling bonds among the nanotubes. The defects and dangling bonds can mediate covalent bonds (such as carbon-carbon bonds) between adjacent nanotubes. These bonds serve as molecular junctions that fuse or weld the nanotubes together into a continuous matrix, thereby enhancing the strength and toughness of the mat, while allowing the mat to be flexible. As a result, the thickness of membrane 26 can be reduced without compromising performance. The reduced thickness, in turn, reduces the profile of the filter, thereby increasing its accessibility to relatively narrow body vessels. The formation of the inter-nanotube bonds can also help keep the bucky paper in the shape that it is in during bond formation. Thus, membrane 26 can be folded or compacted during delivery, and subsequently be returned to its shape during bond formation. This shape recovery can be useful when membrane 26 or nanotube-containing layer 38 is used, for example, with a stent or a balloon. In some embodiments, only selected portions of the nanotube-containing mat are irradiated. The portions that are not irradiated can be more flexible than the irradiated portions and can correspond to portions that are folded during use.

Irradiation can be performed with ions and/or electrons. The energy of the incident ions or electrons is preferably high enough to penetrate through the closest nanotubes and displace carbon atoms of the next closest nanotubes. The nanotubes can be irradiated, for example, with argon ions of about 0.1 keV to about 3 keV (e.g., from about 0.4 to about 1 keV, such as 100 eV, 200 eV, or 400 eV), with irradiation doses from about 5 x 10¹⁵ to about 3 x 10¹⁶ Ar/cm, depending, for example, on the diameters of the nanotubes. The nanotubes can also be irradiated with energetic electrons, e.g., up to 1.25 MeV. In some embodiments, the energy of the incident radiation can be chosen to penetrate the entire thickness of the bucky paper, e.g., by using carbon ions (e.g., C³⁺) with energies of 10 MeV. Irradiation can be performed at room temperature or at elevated temperatures, such as about 800°C, to facilitate migration and annealing of the defects. The ion flux can be from about 8.2 x 10¹³ to about 4.9 x 10¹⁴ Ar/cm²s, and the irradiation time can be from about one minute to about six minutes. In some embodiments, the prior to irradiation, the nanotubes can be annealed at about 900°C for about 30 minutes or at about 470°C for about 50 minutes in air, e.g., to burn off carbonaceous residues and purify the nanotubes. Irradiation of nanotubes is further described, for example, in Krasheninnikov et al., Phys. Rev. B, 66, 245403 (2002); Krasheninnikov et al., Phys. Rev. B 63, 245405 (2001); and Krasheninnikov et al., Krasheninnikov et al., Phys. Rev .B 65, 165423 (2002).

After the inter-nanotubes bonds are created, additional mats of nanotubes can be formed on the first mat to increase the thickness of layer 38 (step 56). Additional layers can be formed by filtering the nanotube-containing mixture over the first mat (steps 48 and 50), removing any residual solvent (step 52), and forming inter-nanotube bonds in the newly formed layers (step 54), as described above. The final thickness of layer 38 can be a function of the specific medical device in which the layer is incorporated. The formed nanotube-containing layer 38 can be peeled away from the filter.

Still referring to Fig. 4, in some embodiments, the nanotubes in layers 38 are modified to enhance interactions between the nanotubes and polymer layers 40 (step 58). The nanotubes can be chemically modified with one or more functional groups that increase interactions with polymer layer 40, e.g., to enhance compatibility or adhesion. For example, the nanotubes can be chemically treated to include carboxylic acid and/or amine chemical moieties, which can interact well with polymers. Functionalization of carbon nanotubes are described, for example, in Bahr et al., J. Am. Chem. Soc. 2001, 123, 6536-6542, Hamon et al., Adv. Mater. 1999, 11, No. 10, 834-840, and U.S. Patent Application Publication 2003/0093107. Alternatively or in addition, the nanotubes can be wrapped with a polymer, such as polyvinyl pyrrolidone (PVP) and/or polystyrene sulfonate (PSS), to enhance compatibility with polymer layers 40 and/or to enhance solubility (e.g., in water). Polymer wrapping of single-walled carbon nanotubes is described in O'Connell et al., Chemical Physics Letters 342 (2001) 265-271. Modification(s) of the nanotubes can be performed before and/or after bonding of the nanotubes.

After a predetermined number of nanotube-containing layers 38 are formed and placed together, polymer layers 40 are formed over the nanotube-containing layers 38 to yield membrane 26 (step 60). Polymer layers 40 can include materials used in medical devices, for example, thermoplastics and thermosets. Examples of thermoplastics include polyolefins, polyamides, such as nylon 12, nylon 11, nylon 6/12, nylon 6, and nylon 66, polyesters, polyethers, polyurethanes, polyureas, polyvinyls, polyacrylics, fluoropolymers, copolymers and block copolymers thereof, such as block copolymers of polyether and polyamide, e.g., Pebax®; and mixtures thereof. Examples of thermosets include elastomers such as EPDM, epichlorohydrin, nitrile butadiene elastomers, silicones, etc. Thermosets, such as expoxies and isocyanates, can also be used. Biocompatible thermosets may also be used, and these include, for example, biodegradable polycaprolactone, poly(dimethylsiloxane) containing polyurethanes and ureas, and polysiloxanes. Polymer layers 40 can also include photocurable resins, such as those used in the dental field, e.g., bisphenol-A-glycidyldimethacrylate (Bis-GMA), triethylenglycol-dimethacrylate (TEGDMA), urethane-dimethacrylate (UDMA), and polycarbonate dimethacrylate, (PCDMA). The photocurable resin can be cured during irradiation of the nanotubes. Other polymers are described in commonly assigned U.S.S.N. 10/645,055, filed August 21, 2003. Mixture 37 can include one or more polymers 40. Polymer layers 40 can be formed by injection molding, casting, spraying, and/or micro-drop techniques.

In some embodiments, polymer layers 40 can include one or more additives. For example, polymer layers 40 can include one or more coupling or compatibilizing agents, dispersants, stabilizers, plasticizers, surfactants, and/or pigments, that enhance interactions between the nanotubes and the polymer. Examples of additive(s) are described in U.S. Patent Application Publication 2003/0093107. Alternatively or in addition, polymer layers 40 can include carbon nanotubes, for example, to enhance the strength of the polymer layers. The nanotubes can be bonded together or not bonded together. Polymer layers 40 can include from about 0.1% to about 60% of nanotubes by weight. Polymer layers 40 can be loaded with a high concentration of nanotubes using a layer-by-layer method described below. Methods of making nanotube-containing mixtures are described, for example, in Biercuk, et al., Applied Physics Letters, 80, 2767 (2002); and Sandler et al., Mat. Res. Soc. Symp. Proc. Vol. 706, 2002 Z4.7.1-Z4.7.6.

In some embodiments, one or more polymer layers 40 include one or more releasable therapeutic agents or a pharmaceutically active compounds, such as described in U.S. Patent No. 5,674,242, and commonly-assigned U.S.S.N. 09/895,415, filed July 2, 2001. The therapeutic agents or pharmaceutically active compounds can include, for example, anti-thrombogenic agents, antioxidants, anti-inflammatory agents, anesthetic agents, anti-coagulants, and/or antibiotics. A specific example includes heparin, which can reduce thrombus formation on the surface of the medical device, particularly long term implants such as a septal defect device or a pulmonary filter.

Other methods of forming membrane 26 include a layer-by-layer technique in which a multilayer structure is formed having a plurality of alternating, oppositely charged layers, as described in U.S.S.N. 10/849,742, entitled "Layer-by-Layer Assembly of Multilayer Regions for Medical Devices" and filed on the same day as this application. The structure can include, for example, a plurality of layers containing charged nanoparticles alternating with a plurality of layers containing charged polyelectrolytes. Charging can be provided, for example, using an electrical potential, by covalently attaching functional groups, and/or by exposing the layers to one or more charged amphiphilic substances. Exemplary materials and techniques are described in U.S.S.N. 10/849,742.

The formed membrane 26 can then be used to form filter 20. For example, membrane 26 can be folded over an appropriately shaped template, such as a conical mandrel, and opposing edges of the membrane can be secured, for example, with an adhesive. Membrane 26 can be attached to frame 24 by solvent casting methods (e.g., wherein liquid membrane polymer is dipped over the frame and allowed to cure and solidify), or by adhesive (such as a cyanoacrylates). Openings 28 can be formed, for example, using excimer laser or other ablation techniques, as described in Weber, U.S. Patent No. 6,517,888. The fusing of the nanotubes can hold the nanotubes together, e.g., so that they are not flushed out through openings 28.

Membrane 26 consists ofnanotube-containing layer(s) 38 and includes polymer layers 40. The interconnections among the nanotubes can enhance the strength of the membrane and allow the nanotubes to withstand forces, e.g., fluid flow, within the body.

Use of intravascular filter 20 is described, for example, in Daniel et al., U.S. Patent No. 6,171,327.

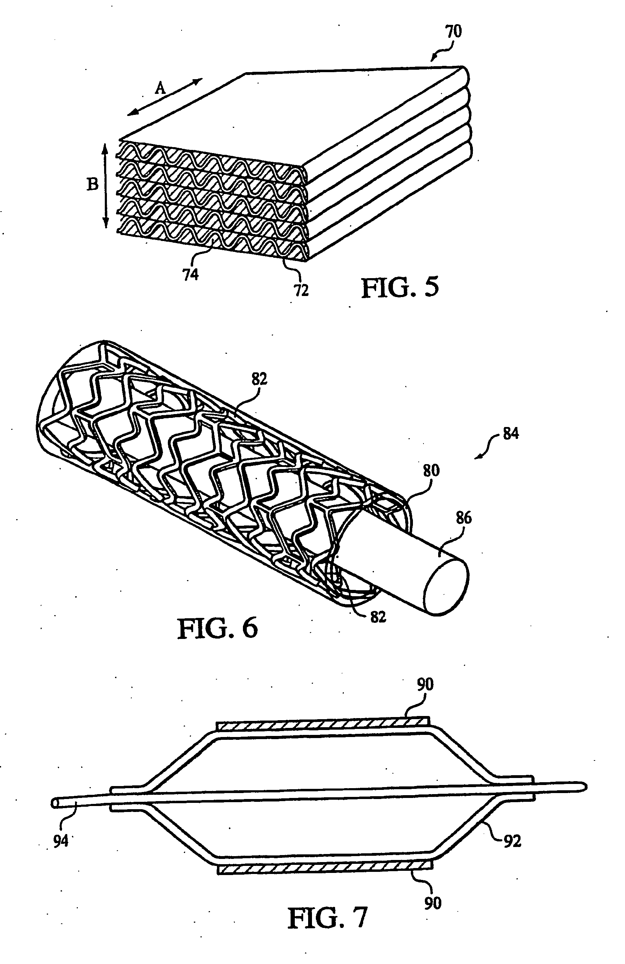

Other embodiments of nanotube-containing layer 38 and membrane 26 can be formed. Referring to Fig. 5, a membrane 70 includes a plurality of corrugated nanotube-containing layers 72 and polymer 74. Nanotube-containing layers 72 can be formed by filtering a mixture containing nanotubes through a corrugated filter, similar to the procedure described above. The corrugation of layers 72 provides membrane 70 with anisotropic mechanical properties such that, for example, the membrane is more crush resistant in direction A than in direction B. Membrane 70 can be formed similar to the methods described above, for example, by spraying, dipping, and/or injection molding polymer 74 between nanotube-containing layers 72.

In certain embodiments, the nanotubes can be aligned, for example, prior to inter-nanotube bond formation. Aligning the nanotubes can be used to control the porosity of the nanotube-containing layers. Aligning the nanotubes, e.g., parallel to each other, can also efficiently pack the nanotubes, thereby increasing the density of the nanotube-containing layer and increasing the likelihood of inter-nanotube formation. Aligning the nanotubes can further enhance the homogeneity of the nanotube-containing layer, which can reduce the occurrence of localized defective or weak spots. The nanotubes can be aligned magnetically. For example, to form a nanotube-containing layer, a mixture containing the nanotubes can be placed on a filter that is not under vacuum but exposed to a magnetic field. The magnetic field, e.g., from about one to about twenty Tesla, is capable of aligning the nanotubes while the nanotubes are dispersed in the solvent above the filter. A vacuum is then applied across the filter to separate the solvent from the aligned nanotubes to form an aligned bucky paper. Magnetic alignment of nanotubes is described, for example, in Choi et al., J. of Appl. Phys., Vol. 94, No. 9, 1 November 2003, 6034-6039. In some embodiments, the magnetic alignment of nanotubes changes, e.g., is reoriented in the major plane of the layer, from one layer to an adjacent layer (e.g., by about 90 degrees).

Embodiments of the membranes or nanotube-containing layers described above can also be used in other medical devices.

For example, embodiments of the membrane or nanotube-containing layer can be formed into a cylindrical tube that can be used as a strong, thin and flexible synthetic vascular graft. The graft can be used to replace a damaged or dysfunctional body vessel (e.g., at the site of an aneurysm or an occlusion), to bypass or divert blood flow around a damaged region, or to create a shunt between an artery and a vein (e.g., for multiple needle access for hemodialysis access). Vascular grafts are described, for example, in U.S. Patent No. 5,320,100.

Referring to Fig. 6, a cylindrical tube of the membrane or the nanotube-containing layer 80 can be used as a strong, thin and flexible graft with a stent 82 to form a stent-graft 84, or a covered stent (as shown, on a support 86 such as a catheter shaft or a balloon catheter). The thinness of the membrane or the nanotube-containing layer reduces resistance to blood flow in the vessel. In some embodiments, one or more of the polymer layers, such as the outermost polymer layer, can include a releasable therapeutic agent or a pharmaceutically active compound, such as described in U.S. Patent No. 5,674,242, commonly-assigned U.S.S.N. 09/895,415, filed July 2, 2001, and U.S.S.N 10/112,391, filed March 28, 2002. The therapeutic agents or pharmaceutically active compounds can include, for example, anti-thrombogenic agents, antioxidants, anti-inflammatory agents, anesthetic agents, anti-coagulants, and antibiotics. The polymer layer(s) can include, for example, a biocompatible, non-porous or semi-porous polymer matrix made of polytetrafluoroethylene (PTFE), expanded PTFE, polyethylene, urethane, or polypropylene.

Stent 82 can be of any desired shape and size (e.g., coronary stents, aortic stents, peripheral stents, gastrointestinal stents, urology stents, and neurology stents). Depending on the application, stent 82 can have an expanded diameter of between, for example, 1 mm to 46 mm. In certain embodiments, a coronary stent can have an expanded diameter of from about 2 mm to about 6 mm. In some embodiments, a peripheral stent can have an expanded diameter of from about 5 mm to about 24 mm. In certain embodiments, a gastrointestinal and/or urology stent can have an expanded diameter of from about 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have an expanded diameter from about 20 mm to about 46 mm. Stent 82 can be balloon-expandable, self-expandable, or a combination of both (e.g., as described in U.S. Patent No. 5,366,504).

Stent-graft 84 can be used, e.g., delivered and expanded, using a balloon catheter system. Suitable catheter systems are described in, for example, Wang U.S. 5,195,969, and Hamlin U.S. 5,270,086. Suitable stents and stent delivery are also exemplified by the NIR on Ranger® system, available from Boston Scientific Scimed, Maple Grove, MN.

Embodiments of the membranes or nanotube-containing layers can be incorporated into a medical balloon. For example, referring to Fig. 7, a tube of the membrane or nanotube-containing layer 90 can be placed over a balloon 92 to serve as a strong, flexible, thin and non-compliant constraint. The tube can be placed over the balloon, for example, as a preformed sleeve, or wrapped around the balloon as a sheet and joined at opposing edges. The structure of the balloon itself can include nanotubes, for example, by forming embodiments of membrane 26 described herein and shaping the membrane into the balloon. Balloon 92 is carried by a catheter shaft 94, which, as described below, can include nanotubes.

In some embodiments, the membrane or nanotube-containing layer can also be formed into the shape of a medical balloon to completely encapsulate the balloon. For example, the membrane or nanotube-containing layer can be formed on a dissolvable (e.g., water soluble) substrate shaped as a medical balloon, and the substrate can be subsequently removed. Prior to applying a nanotube-containing mixture to the substrate, a mist can be sprayed over the substrate to dissolve the outer layer of the substrate and make it sticky. A mixture containing nanotubes can be applied, e.g., sprayed on the substrate to form a layer. After a desired thickness of nanotubes is formed, the nanotubes can be irradiated as described above and the substrate can be dissolved. An example of a dissolvable substrate is degradable polyvinyl alcohol, described, for example, in Cooper et al., Proceedings of the 8th Annual Global Plastics Environmental Conference, Society of Plastics Engineers, Detroit MI, 360, 14 February 2002.

Balloon catheter systems are described, for example, in Wang, U.S. 5,915,969; Hamlin, U.S. 5,270,086; and exemplified by the Maverick® or Symbiot® catheter systems available from Boston Scientific Corp.-Scimed Life Systems, Inc. (Maple Grove, MN).

Embodiments of the membranes or nanotube-containing layers can be sized and shaped to form a variety of catheters. Examples of catheters include guide catheters (e.g., as described in U.S. 6,595,952), tumor ablation catheters, aneurysm catheters, urology catheters, and perfusion catheters (e.g., as described in U.S. 6,503,224). The tube can be formed into an introducer sheath or a restraining sheath for a stent delivery system, for example, as described in U.S. Patent No. 6,488,694 and Raeder-Devens et al., US 2003/0050686. The catheters can include one or more nanotube-containing layers for strength, and one or more layers carrying a marker for fluoroscopic, ultrasound, and/or magnetic resonance detection, as described, for example, in commonly assigned U.S.S.N. 10/390,202, filed March 17, 2003.

The nanotubes having inter-nanotube bonds can also be incorporated in bone cements. For example, the nanotubes can be blended with polymethylmethacrylate (PMMA), bisphenol A diglycerol ether dimethacrylate, triethylene glycol dimethacrylate (TEGDMA), poly(ethylene glycol) methacrylate (PEGMA), *N*,N-dimethyl-p-toluidine, strontium-containing hydroxylapatite, and/or fumed silica. The nanotubes can enhance the strength of the polymers.

Other embodiments are within the claims.

## Claims

1. A method of making a medical device (20) that includes a structure (26) including carbon nanotubes, the method (46) of making the structure comprising:
providing a mixture containing nanotubes (42),
forming a layer (38) containing nanotubes;
forming covalent bonds between carbon nanotubes;
forming polymer layers (40) on nanotube-containing layers (38);
and incorporating the structure including nanotubes into the medical device.

2. The method of claim 1, wherein the structure is a membrane (26).

3. The method of claim 1, further comprising removing solvent from the mixture.

4. The method of claim 1, further comprising repeating the steps of forming a layer, optionally removing the solvent, and forming bonds between carbon nanotubes.

5. The method of claim 1, further comprising modifying the nanotubes.

6. The method of claim 1, wherein the bonds consist essentially of carbon atoms.

7. The method of claim 1, wherein forming bonds comprises irradiating the carbon nanotubes.

8. The method of claim 7, wherein the carbon nanotubes are irradiated with ions or electrons.

9. The method of claim 1, further comprising contacting the nanotubes with a polymer.

10. The method of claim 1, further comprising functionalizing the nanotubes.

11. The method of claim 1, further comprising aligning the nanotubes.

12. The method of claim 11, wherein the nanotubes are aligned magnetically.

13. The method of claim 1, further comprising wrapping a plurality of nanotubes with polymer.

14. The method of claim 1, wherein the nanotubes comprise single-walled carbon nanotubes.

15. A medical device (20) that includes a structure (26) including carbon nanotubes (42), the structure comprising
a layer (40) comprising a polymer; **characterized by**
a layer (38) comprising carbon nanotubes (42) covalently bonded with other carbon nanotubes (42).

16. The device of claim 15, wherein the structure is a membrane (26).

17. The device of claim 15, wherein the carbon nanotubes are bonded by a bond consisting essentially of carbon atoms.

18. The device of claim 15, wherein the layer comprising carbon nanotubes is corrugated.

19. The device of claim 15, comprising a carbon nanotube wrapped with a polymer.

20. The device of claim 15, wherein the carbon nanotubes are aligned.

21. The device of claim 15, the carbon nanotubes comprise an organic functional group bonded to the nanotubes.

22. The device of claim 15, wherein the carbon nanotubes comprise single-walled carbon nanotubes.

23. The device of claim 15, wherein the device is an intravascular filter.

24. The device of claim 15, wherein the devices is a medical balloon.

25. The device of claim 15, wherein the device is a stent graft.

26. The device of claim 15, wherein the device is a catheter or a catheter sheath.

## Patentansprüche

1. Verfahren zum Herstellen einer medizinischen Vorrichtung (20), die eine Struktur (26) beinhaltend Kohlenstoff-Nanoröhren beinhaltet, wobei das Verfahren (46) zum Herstellen der Struktur umfasst:
Bereitstellen einer Mischung, die Nanoröhren (42) beinhaltet;
Formen einer Schicht (3 8), die Nanoröhren beinhaltet;
Formen kovalenter Bindungen zwischen den Kohlenstoff-Nanoröhren;
Formen von Polymerschichten (40) auf den Nanoröhren beinhaltenden Schichten (3 8);
und Einbauen der Struktur, die Nanoröhren beinhaltet, in die medizinische Vorrichtung.

2. Das Verfahren nach Anspruch 1, wobei die Struktur eine Membran (26) ist.

3. Das Verfahren nach Anspruch 1, weiter umfassend Entfernen von Lösungsmittel aus der Mischung.

4. Das Verfahren nach Anspruch 1, weiter umfassend Wiederholen der Schritte des Formens einer Schicht, optional Entfernen des Lösungsmittels, und Formen von Bindungen zwischen den Kohlenstoff-Nanoröhren.

5. Das Verfahren nach Anspruch 1, weiter umfassend Modifizieren der Nanoröhren.

6. Das Verfahren nach Anspruch 1, wobei die Bindungen im Wesentlichen aus Kohlenstoffatomen bestehen.

7. Das Verfahren nach Anspruch 1, wobei das Formen der Bindungen Bestrahlen der Kohlenstoff-Nanoröhren umfasst.

8. Das Verfahren nach Anspruch 7, wobei die Kohlenstoff-Nanoröhren mit Ionen oder Elektronen bestrahlt werden.

9. Das Verfahren nach Anspruch 1, weiter umfassend Kontaktieren mit einem Polymer.

10. Das Verfahren nach Anspruch 1, weiter umfassend Funktionalisieren der Nanoröhren.

11. Das Verfahren nach Anspruch 1, weiter umfassend Ausrichten der Nanoröhren.

12. Das Verfahren nach Anspruch 11, wobei die Nanoröhren magnetisch ausgerichtet werden.

13. Das Verfahren nach Anspruch 1, weiter umfassend Einhüllen der Mehrzahl der Nanoröhren mit Polymer.

14. Das Verfahren nach Anspruch 1, wobei die Nanoröhren einwandige Kohlenstoff-Nanoröhren umfassen.

15. Eine Vorrichtung (20) die eine Struktur (26) beinhaltend Kohlenstoff-Nanoröhren (42) beinhaltet, umfassend
eine Schicht (40) umfassend ein Polymer; **gekennzeichnet durch**
eine Schicht (38) umfassend Kohlenstoff-Nanoröhren (42) kovalent gebunden mit anderen Kohlenstoff-Nanoröhren (42).

16. Die Vorrichtung nach Anspruch 15, wobei die Struktur eine Membran (26) ist.

17. Die Vorrichtung nach Anspruch 15, wobei die Kohlenstoff-Nanoröhren durch eine Bindung verbunden sind, die im Wesentlichen aus Kohlenstoffatomen besteht.

18. Die Vorrichtung nach Anspruch 15, wobei die Schicht umfassend Kohlenstoff-Nanoröhren zerfurcht ist.

19. Die Vorrichtung nach Anspruch 15, umfassend eine Kohlenstoff-Nanoröhre umhüllt mit einem Polymer.

20. Die Vorrichtung nach Anspruch 15, wobei die Kohlenstoff-Nanoröhren ausgerichtet sind.

21. Die Vorrichtung nach Anspruch 15, wobei die Kohlenstoff-Nanoröhren eine an die Nanoröhren gebundene organische funktionelle Gruppe umfasst.

22. Die Vorrichtung nach Anspruch 15, wobei die Kohlenstoff-Nanoröhren einwandige Kohlenstoff-Nanoröhren umfassen

23. Die Vorrichtung nach Anspruch 15, wobei die Vorrichtung ein intravaskulärer Filter ist.

24. Die Vorrichtung nach Anspruch 15, wobei die Vorrichtung ein medizinischer Ballon ist.

25. Die Vorrichtung nach Anspruch 15, wobei die Vorrichtung ein Stent-Graft ist.

26. Die Vorrichtung nach Anspruch 15, wobei die Vorrichtung ein Katheter oder eine Katheterhülle ist.

## Revendications

1. Procédé de fabrication d'un dispositif médical (20) qui contient une structure (26) contenant des nanotubes de carbone, le procédé (46) de fabrication de la structure comportant les étapes qui consistent à :
prévoir un mélange contenant des nanotubes (42),
former une couche (38) contenant des nanotubes,
former des liaisons covalentes entre les nanotubes de carbone,
former des couches polymères (40) sur les couches (38) contenant des nanotubes et
incorporer la structure contenant les nanotubes dans le dispositif médical.

2. Procédé selon la revendication 1, dans lequel la structure est une membrane (26).

3. Procédé selon la revendication 1, comportant en outre l'étape consistant à retirer le solvant du mélange.

4. Procédé selon la revendication 1, comportant en outre la répétition des étapes de formation d'une couche et facultativement d'enlèvement du solvant et de formation de liaison entre les nanotubes de carbone.

5. Procédé selon la revendication 1, comportant en outre l'étape consistant à modifier les nanotubes.

6. Procédé selon la revendication 1, dans lequel les liaisons sont constituées essentiellement d'atomes de carbone.

7. Procédé selon la revendication 1, dans lequel la formation des liaisons comprend l'irradiation des nanotubes de carbone.

8. Procédé selon la revendication 7, dans lequel les nanotubes de carbone sont irradiés par des ions ou des électrons.

9. Procédé selon la revendication 1, comportant en outre l'étape consistant à mettre en contact les nanotubes avec un polymère.

10. Procédé selon la revendication 1, comportant en outre l'étape consistant à fonctionnaliser les nanotubes.

11. Procédé selon la revendication 1, comportant en outre l'étape consistant à aligner les nanotubes.

12. Procédé selon la revendication 11, dans lequel les nanotubes sont alignés magnétiquement.

13. Procédé selon la revendication 1, comportant en outre l'étape consistant à emballer plusieurs nanotubes par un polymère.

14. Procédé selon la revendication 1, dans lequel les nanotubes comprennent des nanotubes de carbone à paroi simple.

15. Dispositif médical (20) contenant une structure (26) qui inclut des nanotubes de carbone (42), la structure comprenant :
une couche (40) comportant un polymère,
**caractérisé par**
une couche (38) comprenant des nanotubes de carbone (42) liés par covalence à d'autres nanotubes de carbone (42).

16. Dispositif selon la revendication 15, dans lequel la structure est une membrane (26).

17. Dispositif selon la revendication 15, dans lequel les nanotubes de carbone sont liés par une liaison constituée essentiellement d'atomes de carbone.

18. Dispositif selon la revendication 15, dans lequel la couche comprenant des nanotubes est ondulée.

19. Dispositif selon la revendication 15, comprenant un nanotube de carbone emballé par un polymère.

20. Dispositif selon la revendication 15, dans lequel les nanotubes de carbone sont alignés.

21. Dispositif selon la revendication 15, dans lequel les nanotubes de carbone comprennent un groupe fonctionnel organique lié aux nanotubes.

22. Dispositif selon la revendication 15, dans lequel les nanotubes de carbone comprennent des nanotubes de carbone à paroi simple.

23. Dispositif selon la revendication 15, dans lequel le dispositif est un filtre intravasculaire.

24. Dispositif selon la revendication 15, dans lequel le dispositif est un ballon médical.

25. Dispositif selon la revendication 15, dans lequel le dispositif est un implant intravasculaire.

26. Dispositif selon la revendication 15, dans lequel le dispositif est un cathéter ou un fourreau de cathéter.
